# EUROPÄISCHE PATENTSCHRIFT

(11) **EP 2 570 144 B1**
(45) Veröffentlichungstag und Bekanntmachung des Hinweises auf die Patenterteilung: **01.08.2018**
(21) Anmeldenummer: 12184084.7
(22) Anmeldetag: 12.09.2012
(51) Int. Cl.: A61M 5/30, A61M 5/20, A61M 5/31, A61M 5/315

(54) **Nadelloser Einweginjektor mit Elementen zur Erhöhung der Auslösesicherheit**
Disposable needleless injector with elements for increasing trigger safety
Injecteur sans aiguille à usage unique avec éléments destinés à augmenter la sécurité de déclenchement

(30) Priorität: 19.09.2011 DE 102011113565
(43) Veröffentlichungstag der Anmeldung: 20.03.2013
(73) Patentinhaber: LTS LOHMANN Therapie-Systeme AG, 56626 Andernach (DE)
(72) Erfinder: Wortmann, Uwe, 35037 Marburg (DE); Hadaschik, Roman, 99817 Eisenach (DE)

(56) Entgegenhaltungen:
- WO-A1-2008/089886
- WO-A1-2008/128615
- WO-A1-2010/069470
- DE-A1-102008 048 595

## Beschreibung

Die Erfindung betrifft einen Einweginjektor mit einem mindestens einen Druckstab umfassenden Gehäuse, mit einem sich am Druckstab abstützenden, mittels eines Federenergiespeichers vorbelasteten Kolbenbetätigungsstempel und mit einer den Druckstab sperrenden Auslöseeinheit, wobei mittels eines Verschiebens der Auslöseeinheit längs des Gehäuses der Druckstab den Federenergiespeicher auslösend entsperrbar ist und wobei der Kolbenbetätigungsstempel ein Stößelteil trägt.

Aus der DE 10 2008 048 595 A1 ist ein derartiger Einweginjektor bekannt. Die zusammenwirkenden Flächen des Kolbenbetätigungsstempels und des Druckstabs können mit einer keramischen Panzerung versehen sein. Auf der Seite des Kolbenbetätigungsstempels kann zur Verstärkung eine Unterlegscheibe aufgeklebt sein.

Der vorliegenden Erfindung liegt die Problemstellung zugrunde, einen Einweginjektor mit hoher Auslösseicherheit zu entwickeln.

Diese Problemstellung wird mit den Merkmalen des Hauptanspruchs gelöst. Dazu ist am Druckstab ein Keilteil befestigt. Das Stößelteil und das Keilteil sind Teile eines Keilgetriebes, wobei das Keilteil beim Auslösen des Einweginjektors mittels des Stößelteils das Keilgetriebe auflösend verdrängbar ist.

Weitere Einzelheiten der Erfindung ergeben sich aus den Unteransprüchen und den nachfolgenden Beschreibungen schematisch dargestellter Ausführungsbeispiele.
- Figur 1:: Einweginjektor;
- Figur 2:: Schnittdarstellung des Einmalinjektors vor dem Auslösen und während des Auslösens;
- Figur 3:: Detail von Figur 2 vor dem Auslösen;
- Figur 4:: Stößelteil, rund;
- Figur 5:: Stößelteil, rechteckig;
- Figur 6:: Kolbenbetätigungsstempel mit Führungsnut;
- Figur 7:: Kolbenbetätigungsstempel mit Zentrierflächen;
- Figur 8:: Gehäuse mit Keilteilen;
- Figur 9:: Keilteil mit Außenbefestigung;
- Figur 10:: Keilteil mit seitlicher Befestigung;
- Figur 11:: Keilteil, aufschiebbar;
- Figur 12:: Keilteil, aufrastbar;
- Figur 13:: Keilteil mit klammerartigen Befestigungselementen.

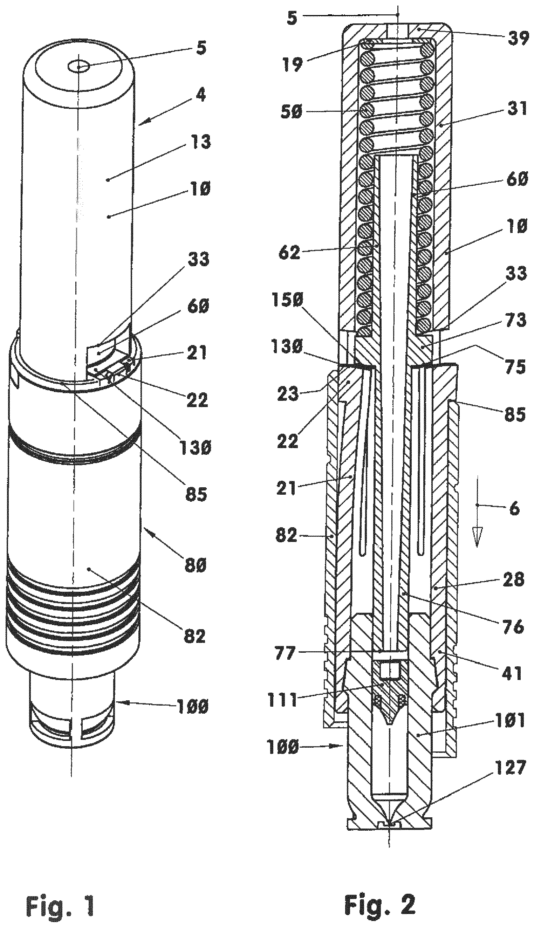

Die Figur 1 zeigt einen Einweginjektor (4). Im einsatzbereiten Zustand umfasst dieser ein Gehäuse (10), eine Auslöseeinheit (80) und eine Zylinder-Kolben-Einheit (100).

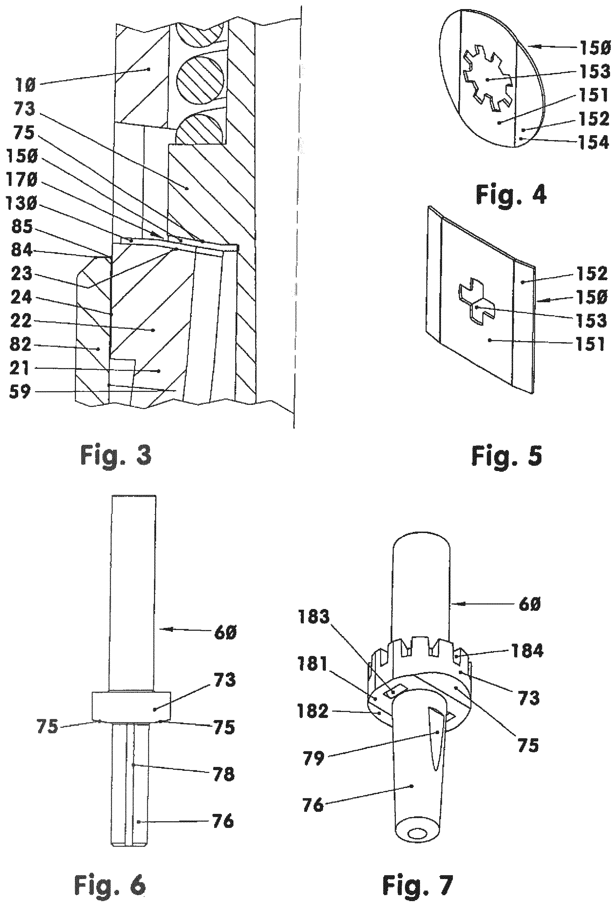

Das einteilige Gehäuse (10) ist topfförmig ausgebildet und hat eine z.B. zylinderförmige Außenfläche (13). Es verfügt im Ausführungsbeispiel über zwei einander gegenüber angeordnete Durchbrüche (33) und über Druckstäbe (21). Die oberen Enden der Druckstäbe (21), die Nocken (22) stützen sich in dem hier dargestellten Zustand des Einweginjektors (4) vor dem Beginn des Auslösens an der Innenseite eines Auslöseelements (82) der Auslöseeinheit (80) ab, vgl. die Figuren 2 und 3. Ein federbelasteter Kolbenbetätigungsstempel (60) stützt sich mittels eines Stößelteils (150) und eines Keilteils (130) an den Druckstäben (21) des Gehäuses (10) ab.

Das Auslöseelement (82) ist hülsenartig ausgebildet und umgibt längsverschieblich den unteren Bereich der z.B. zylindrischen Außenfläche (13) des Gehäuses (10). Im Ausführungsbeispiel hat das Auslöseelement (82) eine kreisringförmige Querschnittsfläche. Die Querschnittsflächen des Gehäuses (10) und des Auslöseelements (82) können auch durch ein Rechteck, Sechseck, eine Ellipse, etc. begrenzt sein.

Die Zylinder-Kolben-Einheit (100) sitzt im unteren Bereich des Gehäuses (10) und ragt aus diesem heraus.

Die Figur 2 zeigt einen Längsschnitt eines Injektors (4). Die linke Seite der Schnittdarstellung zeigt den Injektor (4) vor der Betätigung der Auslöseeinheit (80). Auf der rechten Seite ist das Auslöseelement (82) in der Auslösebewegungsrichtung (6), also nach unten, verschoben.

Das Gehäuse (10) ist ein unten offener Hohlkörper mit obenliegendem Boden (39). Es wird z.B. aus einem glasfaserverstärkten Polyamid durch Spritzgießen gefertigt. Das Gehäuse (10) hat eine weitgehend rohrförmige Gestalt und ist in zwei Funktionsbereiche aufgeteilt, das ist zum einen der obere Mantelbereich (31) und zum anderen der untere Fixierbereich (41). Im Gehäuse (10) sind ein Kolbenbetätigungsstempel (60) und eine Schraubendruckfeder (50) als Federenergiespeicher angeordnet.

Am unteren Rand des einzelnen Durchbruchs (33) ist jeweils ein Druckstab (21) als elastischer Biegebalken angeformt. Die Anformstelle für die Druckstäbe (21) liegt knapp oberhalb des Fixierbereichs (41). Zur Ausbildung des jeweiligen Druckstabs (21) befindet sich im unteren Bereich des Mantelabschnitts (31) ein schmaler, zumindest annähernd u-förmiger Spalt, der den einzelnen Druckstab (21) seitlich und oben umgibt.

Der Druckstab (21) hat beispielsweise auf 80% seiner Länge die Wandstärke und die Krümmung der Wandung des Gehäuses (10). Dieser Bereich hat unter anderem auch die Funktion eines federelastischen Biegebalkens (28). Er hat einen sichelförmigen Querschnitt.

Ggf. kann ein Teil dieses Biegebalkens (28) auch mit einem rechteckigen Querschnitt ausgestattet sein, um bei der Nutzung auftretende Biegespannungen im Biegebalkenrandbereich zu reduzieren.

Bei Injektoren, bei denen der Kolbenbetätigungsstempel (60) im Gehäuse (10) - zumindest abschnittsweise - mit geringem Spiel geradgeführt ist und der Kolbenbetätigungsstempel (60) eine ausreichende Biegefestigkeit aufweist, kann anstatt zweier oder mehrerer Druckstäbe (21) auch nur ein einziger Druckstab (21) verwendet werden.

Das hier obere Ende des einzelnen Druckstabs (21) wird durch den radial nach außen abstehenden Nocken (22) gebildet. Letzterer hat zumindest eine in Richtung der Mittellinie (5) orientierte Abstützfläche (23) und eine der Mittellinie (5) abgewandte Anlagefläche (24), vgl. Figur 3.

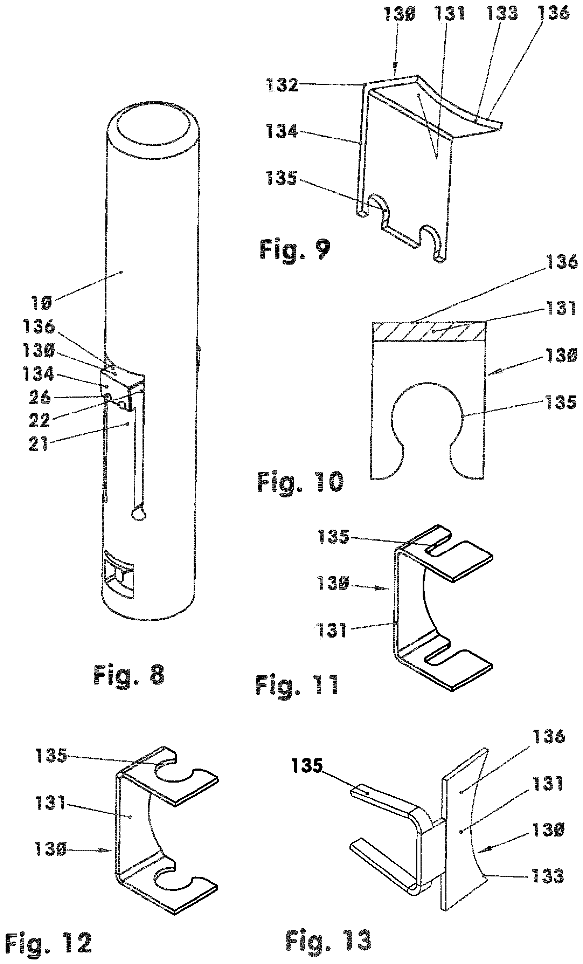

Auf dem Druckstab (21) sitzt das Keilteil (130), beispielsweise ein Gleitschuh (130), vgl. z.B. Figur 8. Dieser Gleitschuh (130) übergreift die Abstützfläche (23) des Druckstabs (21) und ist beispielsweise an der nach außen zeigenden Anlagefläche (24) des Nockens (22) befestigt. Der Gleitschuh (130) verhindert, dass sich der Kolbenbetätigungsstempel (60) in den Druckstab (21) einarbeitet.

Die Figur 9 zeigt einen derartigen Gleitschuh (130). Er ist aus einem abgewinkelten Blech hergestellt. Der Werkstoff des Gleitschuhs (130) ist beispielsweise ein austenitischer Stahl, der chemisch inert und physikalisch beständig ist. Auch der Einsatz von Aluminium oder eines anderen metallischen Werkstoffs ist denkbar.

Der auf der Abstützfläche (23) aufliegende Keilschenkel (131) des Gleitschuhs (130) ist im Ausführungsbeispiel eben ausgebildet. Er hat eine gerade Außenkante (132) und eine zylinderabschnittsförmige Innenflanke (133). Die gedachte Mittellinie des Zylinderabschnitts fällt mit der Mittellinie (5) des Injektors (4) zusammen.

Der Außenschenkel (134) des Gleitschuhs (130) liegt an der z.B. planen Anlagefläche (24) an. Er trägt zwei Aufnahmeöffnungen (135), die mit auf der Anlagefläche (24) angeordneten Zapfen (26) verrastbar sind.

Im unteren Bereich des Gehäuses (10) befinden sich Halteelemente zur Befestigung der Zylinder-Kolben-Einheit (100). Die Zylinder-Kolben-Einheit (100) besteht im Ausführungsbeispiel aus einem, mit einer Injektionslösung befüllbaren, transparenten Zylinder (101). In der Darstellung der Figur 2 sitzt ein Kolben (111) in der hinteren Position. Oberhalb des Kolbens (111) ist im Gehäuse (10) der Kolbenbetätigungsstempel (60) z.B. so angeordnet, dass er den Kolben (111) zwar nicht berührt, jedoch mit seinem unteren Ende z.B. im oberen Bereich des Zylinders (101) seitlich geführt wird.

Das Auslöseelement (82) ist z.B. aus Acrylnitril-Butadien-Styrol-Copolymer (ABS) hergestellt. Es endet rückwärtig mit einer scharfen Kante (85), die Teil einer stirnseitigen Rücksprungflanke (84) des Auslöseelements (82) ist. Unterhalb der Kante (85) berühren in der Darstellung der Figur 3 die Druckstäbe (21) angeformte Nocken (22) mit ihren außen liegenden Anlageflächen (24) sichernd die Innenwandung (59) des Auslöseelements (82).

Der im Gehäuse (10) angeordnete Kolbenbetätigungsstempel (60) ist in zwei Bereiche aufgeteilt. Der untere Bereich ist der Kolbenschieber (76). Sein Durchmesser ist etwas kleiner als der Innendurchmesser des hinteren Bereichs des Zylinders (101). Die untere Stirnfläche des Kolbenschiebers (76) wirkt direkt auf den Kolben (111).

Der obere Bereich des Kolbenbetätigungsstempels (60), der Stempelteller (73), ist eine flache, zumindest bereichsweise zylindrische Scheibe, deren Außendurchmesser einige Zehntel Millimeter kleiner ist als der Innendurchmesser des Gehäuses (10) im Mantelbereich (31). Die untere Stirnseite weist eine um den Kolbenschieber (76) herum angeordnete Bundfläche (75) auf. Sie hat beispielsweise die Form eines Kegelstumpfmantels, dessen Spitzenwinkel ca. 100 bis 140 Grad beträgt. Im dargestellten Ausführungsbeispiel hat die Bundfläche (75) einen Spitzenwinkel von 140 Grad. Die gedachte Spitze des Kegelstumpfmantels liegt auf der Mittellinie (5) im Bereich des Kolbenschiebers (76). Die Bundfläche (75) kann sphärisch gekrümmt sein, sie kann einzelne, schräg zueinander angeordnete Ebenen aufweisen, etc.

Der Kolbenschieber (76) kann selbstverständlich auch als separates, vom Stempelteller (73) getrenntes, Bauteil ausgeführt sein. Hierzu ist er dann an der Innenwandung des Gehäuses (10) geführt.

Zwischen dem Stempelteller (73) und dem oben liegenden Boden (39) des Gehäuses (10) sitzt vorgespannt die Schraubendruckfeder (50). Die Schraubendruckfeder (50) stützt sich am oben liegenden Boden (39) des Gehäuses (10) unter Zwischenschaltung einer Distanzhülse (19) ab. Die Federkraft der Schraubendruckfeder (50) wird über den Stempelteller (73) auf die Druckstäbe (21) übertragen. Aufgrund der Neigung der Bundfläche (75) werden die Druckstäbe (21) keilgetriebeartig radial nach außen gedrängt. Die Auslösehülse (82) stützt diese Radialkraft dauerhaft ab.

Der Kolbenbetätigungsstempel (60) hat oberhalb des Stempeltellers (73) einen Führungszapfen (62). Letzterer führt die Schraubendruckfeder (50) oder wird durch diese geführt. Unterhalb des Stempeltellers (73) befindet sich zentral in der Verlängerung des Führungszapfens (62) der Kolbenschieber (76), der bei einer Betätigung des Einmal-Injektors (4) auf den Kolben (111) wirkt. Der Kolbenschieber (76) hat eine Stirnfläche (77), mit der er beim Auslösen die komplementär geformte Stirnfläche des Kolbens (111) kontaktiert.

Der Kolbenbetätigungsstempel (60) trägt das Stößelteil (150), z.B. einen Gleitsattel (150). Ein derartiger Gleitsattel (150) ist beispielsweise in der Figur 4 dargestellt. Er ist z.B. aus dem gleichen Werkstoff wie der Gleitschuh (130) hergestellt. Der Gleitsattel (150) ist in dieser Darstellung eine Scheibe mit einem zentralen Lagerungs- und Zentrierbereich (151) und mit zwei an diesen anschließenden Führungsbereichen (152). Der ebene Lagerungs- und Zentrierbereich (151) hat eine zentrale Ausnehmung (153), die eine Vielkeilnabe bildet. Anstatt einer Vielkeilnabe kann die Ausnehmung (153) z.B. einen einzelnen Keil oder eine kreissegmentförmige Abflachung aufweisen. Die Führungsbereiche (152) schließen mit dem Lagerungs- und Zentrierbereich (151) einen stumpfen Winkel ein. Beispielsweise haben sie die Steigung der Bundfläche (75). Im montierten Zustand, vgl. die Figuren 2 und 3, liegen die Führungsbereiche (152) zwischen der Bundfläche (75) und dem Gleitschuh (130). Gegebenenfalls kann der Gleitsattel (150) am Kolbenbetätigungsstempel (60) befestigt sein.

Bevor der Einweginjektor gefüllt und eingesetzt wird, ist der Federenergiespeicher (50) vorgespannt, vgl. Figur 2, linker Teilschnitt. Die beiden auf Druck belasteten Druckstäbe (21) halten den Kolbenbetätigungsstempel (60) mittels des aus Stößelteil (150) und Keilteil (130) gebildeten Keilgetriebes (170) an dessen Stempelteller (73) in seiner vorgespannten Lage.

Die Biegeelastizität der Druckstäbe (21) und der Stempelteller (73) drücken die Stützstäbe (21) zumindest annähernd radial nach außen gegen das Auslöseelement (82). Dort liegen sie über Nocken (22) und gegebenenfalls über die Gleitschuhe (130) am Auslöseelement (82) an.

Beispielsweise bei einer Ausführung des Stößelteils (150) und des Keilteils (130) aus einem metallischen Werkstoff können mittels des Federenergiespeichers (50) hohe Flächenpressungen auf die Kontaktpaarung aufgebracht werden. Der hohe Elastizitätsmodul des Werkstoffs - dieser ist beispielsweise mindestens zehnmal so hoch wie der Elastizitätsmodull des Gehäusewekstoffs - verhindert zusammen mit einer mittleren Oberflächenqualität u.a. ein Kaltverschweißen der Werkstoffe. Außerdem wird ein Einarbeiten der Druckstäbe (21) in die Bundfläche (75) verhindert.

Um den Einweginjektor benutzen zu können, muss zunächst die Zylinder-Kolben-Einheit (100) befüllt werden. Dazu wird z.B. ein Stopfen aus der Adapteröffnung (127) entfernt und eine Injektionslösung z.B. eingepresst oder eingesaugt. Der Kolben (111) wird hierbei zurückgedrückt oder zurückgezogen.

Nun kann das Auslöseelement (82) in Richtung der Zylinder-Kolben-Einheit (100) verschoben werden, vgl. Figur 2, rechter Teilschnitt. Der in diesem Teilschnitt dargestellte Zustand ist nicht statisch, er wird daher im Folgenden als fiktiver Zustand bezeichnet.

Beim Auslösen des Einweginjektors (4) gleitet das Auslöseelement (82) auf der Außenwandung (13) des Gehäuses (10) in der Auslösebewegungsrichtung (6) linear nach unten, also in Richtung der Injektionsstelle. Der Kolbenbetätigungsstempel (60) verdrängt, angetrieben vom Federelement (50), mittels des Stößelteils (150) das Keilteil (130) zusammen mit dem Druckstab (21). Hierbei bildet die dem Keilteil (130) zugewandte Fläche des Stößelteils (150) eine Stößelfläche (154). Die dem Stößelteil (150) zugewandte Fläche des Keilteils (130) bildet eine Keilfläche (136) des Keilgetriebes (170). Aufgrund der Werkstoffpaarung des Keilgetriebes (170) hat dieses eine geringe Anlaufreibung und eine geringe Gleitreibung. Das Abgleiten des Kolbenbetätigungsstempels (60) von den Stützstäben (21) erfolgt nahezu widerstandsfrei. Der Einweginjektor (4) hat hierdurch eine hohe Auslösesicherheit.

Die Anlageflächen (24) der Druckstäbe (21) bzw. die Außenschenkel (134) der Gleitschuhe (130) rutschen über die Kante (85). Die Druckstäbe (21) biegen sich elastisch nach außen in ihre eigentliche Ausgangslage und springen unter der Kraft des Federelements (50) radial nach außen.

Sobald die Stößelteile (130) die Keilteile (150) vollständig verdrängt haben, die Nocken (22) also in die Aufweitung verdrängt sind, schnellt der Kolbenbetätigungsstempel (60) ungehindert nach unten. Das Keilgetriebe (170) ist nun aufgelöst. Der Kolben (111) wird nach unten gedrückt. Der Zylinder (100) wird entleert.

Die Figur 5 zeigt ein weiteres Beispiel eines Gleitsattels (150). Dieser hat eine rechteckige Grundfläche und dient beispielsweise zum Einsatz in einem Einweginjektor (4) mit rechteckigem Innenquerschnitt. Die zentrale Ausnehmung (153) ist hier kreuzartig gestaltet. Im eingebauten Zustand durchdringt der Kolbenschieber (76) diese Ausnehmung (153). Auch in diesem Ausführungsbeispiel sind die außenliegenden Führungsbereiche (152) geneigt zum zentralen Lagerungs- und Zentrierbereich (151) angeordnet. Gegebenenfalls kann auch dieser Gleitsattel (150) mit dem Kolbenbetätigungsstempel (60) verrastet sein.

In der Figur 6 ist ein Kolbenbetätigungsstempel (60) dargestellt. Der Stempelteller (73) hat zwei Bundflächen (75). Der Kolbenschieber (76) trägt in diesem Ausführungsbeispiel eine Längsnut (78) als Verdrehsicherung. Mittels dieser Längsnut (78) kann auch der Gleitsattel (150) zentriert werden. Statt einer Nut (78) kann der Kolbenschieber auch einen z.B. radial herausstehenden Zapfen aufweisen.

Ein weiteres Beispiel eines Kolbenbetätigungsstempels (60) zeigt die Figur 7. Der konisch ausgebildete Kolbenschieber (76) weist eine Abflachung (79) auf. Hiermit kann beispielsweise ein Verdrehen des Kolbenbetätigungsstempels (60) im Gehäuse (10) verhindert werden. Der Stempelteller (73) hat einen zentralen Bereich (181), an den zwei prismenartige Abschnitte (182) angrenzen. An seiner Unterseite hat der Stempelteller (73) im zentralen Bereich (181) zwei Ausnehmungen (183). In diesen kann ein Gleitsattel (150) gehalten werden. Die der Schraubendruckfeder (50) zugewandte Oberseite des Stempeltellers (73) hat prismenförmige Aussparungen (184).

Die Figur 10 zeigt einen Querschnitt eines Gleitschuhs (130). Dieser ist u-förmig ausgebildet und wird beispielsweise von oben aufgesetzt und mittels der Aufnahmeöffnungen (135) seitlich an dem Nocken (22) des jeweiligen Druckstabs (21) verankert. Beispielsweise ist der Gleitschuh (130) mit dem Druckstab (21) verrastet. Hierzu kann der Nocken (22) z.B. zylindrische Zapfen aufweisen. Der Keilschenkel (131) verhindert ein Verdrehen des Gleitschuhs (130) gegenüber dem Nocken (22). Die obenliegende Keilfläche (136) des Keilschenkels (131) überdeckt nach dem Aufsetzen die Abstützfläche (23) des Druckstabs (21).

In der Figur 11 ist ein weiterer u-förmiger Gleitschuh (130) dargestellt. Dieser wird von Richtung der der Innenseite oder von der Außenseite des Gehäuses (10) auf den Nocken (22) des jeweiligen Druckstabes (21) aufgesetzt. Er kann hierbei mittels der Aufnahmeöffungen (135) z.B. in zwei an den Seitenflächen des Nockens (22) angeordnete Führungsleisten eingesetzt werden. Gegebenenfalls kann der Gleitschuh (130) und/oder der Nocken (22) des Druckstabes (21) bei der Montage elastisch verformt werden. Hierdurch wird beispielsweise ein Verschwenken oder Verschieben des Gleitschuhs (130) gegenüber dem Druckstab (21) nach der Montage verhindert.

Auch der in der Figur 12 dargestellte Gleitschuh (130) ist u-förmig ausgebildet und wird seitlich an den Nocken (22) befestigt. Die Befestigung erfolgt mittels Verrasten. Auch in diesem Fall erfolgt die Montage z.B. bei ausgebogenen Druckstäben (21) aus der Richtung des Gehäuses (10) oder von der Außenseite des Gehäuses (10).

Die Figur 13 zeigt in einer dimetrischen Ansicht ein Keilteil (130) mit klammerartigen Befestigungselementen (135). Dieser Gleitschuh (130) ist beispielsweise von oben an den Nocken (22) aufsteckbar. Hierbei greifen die Befestigungselemente (135) beispielsweise in Aussparungen der Abstützfläche (23) des Nockens (22) ein. Die Keilfläche (136) ist nach innen auch in diesem Ausführungsbeispiel durch die scharfkantig ausgebildete Innenkante (133) begrenzt.

Der Stößel (150) kann am Kolbenbetätigungsstempel (60) kraft- und/oder formschlüssig befestigt sein. Auch eine Fügeverbindung mittels Zusatzstoffen, z.B. eine Klebeverbindung ist zwischen den Bauteilen denkbar. Auch die Fügeverbindung zwischen dem Keil (130) und dem Druckstab (21) kann so ausgebildet sein.

Selbstverständlich ist es auch denkbar, die verschiedenen genannten Ausführungsformen miteinander zu kombinieren.

### Bezugszeichenliste:

- 4: Einweginjektor
- 5: Mittellinie des Injektors, Längsrichtung
- 6: Auslösebewegungsrichtung von (82), Abwärtsbewegung Richtungspfeil

- 10: Gehäuse, einteilig
- 13: Außenfläche, zylindrisch
- 19: Distanzhülse

- 21: Druckstäbe, Stützstäbe; Zughaken
- 22: Nocken
- 23: Abstützfläche
- 24: Anlagefläche
- 26: Zapfen
- 28: Biegebalken

- 31: Mantelbereich
- 33: Durchbrüche
- 39: Boden
- 41: Fixierbereich für die Zylinder-Kolben-Einheit

- 50: Federelement, Schraubendruckfeder, Federenergiespeicher
- 59: Innenwandung von (82)

- 60: Kolbenbetätigungsstempel
- 62: Führungszapfen
- 73: Stempelteller
- 75: Bundfläche, konisch
- 76: Kolbenschieber
- 77: Kolbenschieber-Stirnfläche, kegelmantelförmig
- 78: Längsnut
- 79: Abflachung

- 80: Auslöseeinheit
- 82: Auslöseelement
- 84: Rücksprungflanke
- 85: Kante, scharfkantig

- 100: Zylinder-Kolben-Einheit
- 101: Zylinder

- 111: Kolben

- 127: Adapteröffnung

- 130: Gleitschuh, Keilteil
- 131: Schenkel von (130), Keilschenkel
- 132: Außenkante
- 133: Innenkante
- 134: Außenschenkel
- 135: Aufnahmeöffnungen, Befestigungselemente
- 136: Keilfläche

- 150: Gleitsattel, Stößelteil
- 151: Lagerungs- und Zentrierbereich
- 152: Führungsbereich
- 153: Ausnehmung
- 154: Stößelfläche

- 170: Keilgetriebe

- 181: zentraler Bereich
- 182: schräge Abschnitte
- 183: Ausnehmungen
- 184: Aussparungen.

## Patentansprüche

1. Einweginjektor (4) mit einem mindestens einen Druckstab (21) umfassenden Gehäuse (10), mit einem sich am Druckstab (21) abstützenden, mittels eines Federenergiespeichers (50) vorbelasteten Kolbenbetätigungsstempel (60) und mit einer den Druckstab (21) sperrenden Auslöseeinheit (80), wobei mittels eines Verschiebens der Auslöseeinheit (80) längs des Gehäuses (10) der Druckstab (21) den Federenergiespeicher (50) auslösend entsperrbar ist, wobei der Kolbenbetätigungsstempel (60) ein Stößelteil (150) trägt und wobei der einzelne Druckstab (21) einen Nocken (22) mit einer Abstützfläche (23) umfasst, **dadurch gekennzeichnet,**
- **dass** am Druckstab (21) ein Keilteil (130) verrastet befestigt ist,
- **dass** das Keilteil (130) die Abstützfläche (23) des Druckstabes (21) übergreift und
- **dass** das Stößelteil (150) und das Keilteil (130) Teile eines Keilgetriebes (170) sind, wobei das Keilteil (130) beim Auslösen des Einweginjektors (4) mittels des Stößelteils (150) das Keilgetriebe (170) auflösend verdrängbar ist.

2. Einweginjektor (4) nach Anspruch 1,
**dadurch gekennzeichnet,**
**dass** das Stößelteil (150) eine dem Druckstab (21) zugewandte Stößelfläche (154) aufweist.

3. Einweginjektor (4) nach Anspruch 1,
**dadurch gekennzeichnet,**
**dass** das Stößelteil (150) form-, kraft- und/oder stoffschlüssig am Kolbenbetätigungsstempel (60) befestigt ist.

4. Einweginjektor (4) nach Anspruch 1,
**dadurch gekennzeichnet,**
**dass** das Stößelteil (150) den Kolbenbetätigungsstempel (60) umgibt.

5. Einweginjektor (4) nach Anspruch 1,
**dadurch gekennzeichnet,**
**dass** das Keilteil (130) eine einem Stempelteller (73) des Kolbenbetätigungsstempels (60) zugewandte Keilfläche (136) aufweist.

6. Einweginjektor (4) nach Anspruch 1,
**dadurch gekennzeichnet,**
**dass** das Keilteil (130) form-, kraft- und/oder stoffschlüssig am Druckstab (60) befestigt ist.

7. Einweginjektor (4) nach Anspruch 1,
**dadurch gekennzeichnet,**
**dass** das Stößelteil (150) und/oder das Keilteil (130) aus einem chemisch inerten metallischen Werkstoff besteht.

## Claims

1. Disposable injector (4) having a housing (10) comprising at least one pressure rod (21), having a piston-actuating ram (60) that is preloaded by means of a spring energy store (50), and having a triggering unit (80) locking the pressure rod (21), wherein, by means of the triggering unit (80) being moved along the housing (10), the pressure rod (21) is unlockable, triggering the spring energy store (50), wherein the piston-actuating ram (60) bears a plunger part (150) and wherein the single pressure rod (21) comprises a cam (22) having a supporting face (23), **characterized**
- **in that** a wedge part (130) is fastened to the pressure rod (21) in an interlocking manner,
- **in that** the wedge part (130) engages over the supporting face (23) of the pressure rod (21), and
- **in that** the plunger part (150) and the wedge part (130) are parts of a wedge mechanism (170), wherein the wedge part (130) is displaceable, triggering the wedge mechanism (170), when the disposable injector (4) is triggered by means of the plunger part (150).

2. Disposable injector (4) according to Claim 1,
**characterized**
**in that** the plunger part (150) has a plunger face (154) facing the pressure rod (21).

3. Disposable injector (4) according to Claim 1,
**characterized**
**in that** the plunger part (150) is fastened to the piston-actuating ram (60) in a form-fitting, force-fitting and/or materially bonded manner.

4. Disposable injector (4) according to Claim 1,
**characterized**
**in that** the plunger part (150) surrounds the piston-actuating ram (60).

5. Disposable injector (4) according to Claim 1,
**characterized**
**in that** the wedge part (130) has a wedge face (136) facing a ram plate (73) of the piston-actuating ram (60).

6. Disposable injector (4) according to Claim 1,
**characterized**
**in that** the wedge part (130) is fastened to the pressure rod (60) in a form-fitting, force-fitting and/or materially bonded manner.

7. Disposable injector (4) according to Claim 1,
**characterized**
**in that** the plunger part (150) and/or the wedge part (130) consist(s) of a chemically inert metal material.

## Revendications

1. Injecteur à usage unique (4) avec un boîtier (10) comprenant au moins une barre de poussée (21), avec un tampon d'actionnement de piston (60) prenant appui sur la barre de poussée (21) et pré-chargé au moyen d'un accumulateur d'énergie à ressort (50) et avec une unité de déclenchement (80) bloquant la barre de poussée (21), dans lequel la barre de poussée (21) peut être débloquée en libérant l'accumulateur d'énergie à ressort (50) au moyen d'un déplacement de l'unité de déclenchement (80) le long du boîtier (10), dans lequel le tampon d'actionnement de piston (60) porte une partie de poussoir (150) et dans lequel l'unique barre de poussée (21) comprend une came (22) avec une face d'appui (23), **caractérisé en ce que**
- une partie de coin (130) est fixée par encliquetage sur la barre de poussée (21),
- la partie de coin (130) recouvre la face d'appui (23) de la barre de poussée (21), et
- la partie de poussoir (150) et la partie de coin (130) font partie d'une transmission à coin (170), dans lequel la partie de coin (130) peut être repoussée en déclenchant la transmission à coin (170) lors du déclenchement de l'injecteur à usage unique (4) au moyen de la partie de poussoir (150).

2. Injecteur à usage unique (4) selon la revendication 1, **caractérisé en ce que** la partie de poussoir (150) présente une face de poussoir (154) tournée vers la barre de poussée (21).

3. Injecteur à usage unique (4) selon la revendication 1, **caractérisé en ce que** la partie de poussoir (150) est fixée par emboîtement, par adhérence et/ou matériellement au tampon d'actionnement de piston (60).

4. Injecteur à usage unique (4) selon la revendication 1, **caractérisé en ce que** la partie de poussoir (150) entoure le tampon d'actionnement de piston (60).

5. Injecteur à usage unique (4) selon la revendication 1, **caractérisé en ce que** la partie de coin (130) présente une face de coin (136) tournée vers un collet de tampon (73) du tampon d'actionnement de piston (60).

6. Injecteur à usage unique (4) selon la revendication 1, **caractérisé en ce que** la partie de coin (130) est fixée par emboîtement, par adhérence et/ou matériellement à la barre de poussée (60).

7. Injecteur à usage unique (4) selon la revendication 1, **caractérisé en ce que** la partie de poussoir (150) et/ou la partie de coin (130) se compose d'un matériau métallique chimiquement inerte.
